# EUROPEAN PATENT SPECIFICATION

(11) **EP 3 862 805 B1**
(45) Date of publication and mention of the grant of the patent: **10.01.2024**
(21) Application number: 20164450.7
(22) Date of filing: 20.03.2020
(51) Int. Cl.: G02B 27/00, A61F 9/02, G02C 7/08, G02C 9/04, G02C 11/08

(54) **GOGGLES WITH RETAINING THREAD OF TEAR-OFF LENSES**
BRILLE MIT HALTEGEWINDE FÜR ABREISSLINSEN
LUNETTES DOTÉES D'UN FIL DE RETENUE DE LENTILLES DÉTACHABLES

(30) Priority: 07.02.2020 IT 202000002458
(43) Date of publication of application: 11.08.2021
(73) Proprietor: Plastiche Cassano S.r.l., 21012 Cassano Magnago (VA) (IT)
(72) Inventor: FRANCHI, Augusto, 21012 Cassano Magnago (IT)
(74) Representative: Mittler, Andrea

(56) References cited:
- FR-A1- 3 003 730
- FR-A1- 3 060 953
- US-A- 5 685 022
- US-A1- 2017 071 792

## Description

The present invention relates to goggles with retaining thread of tear-off lenses.

Frontally open helmets to which goggles are coupled are widely used in motocross, and more generally in off-road activities, e.g. with bicycles and quads.

Said goggles consist of a visor and an elastic band to secure the goggles to the helmet and thus to the user.

The use of tear-off lens packages of the type shown in US-2014/0352041 is known in competitions. These are packages which can be applied in a removable manner to the base lens of the visor. Said packages have two sets of lateral through-holes through which attachment means fixed to the base lens of the visor. Said packages may be of the standard type, in which the tear-off lenses are simply superimposed, or of the laminated type, in which the tear-off lenses adhere to each other by means of adhesive.

Advantageously, when the rider has viewing difficulty due to dirt or wear, he can use a simple tab to remove the outermost tear-off lens from the package.

The tear-off lenses are normally made of polycarbonate, generally of non-degradable, and therefore pollutant, plastic material.

As a result of the tearing, the tear-off lens falls to the ground and should, in theory, be recovered at the end of the race by the rider or by the track staff for appropriate disposal.

Disadvantageously, this usually does not happen completely and most of the tear-off lenses remain on the ground, also because they are not very visible.

The motorcycle federation has banned the use of tear-off lenses during motorcycle races because they were abandoned along the tracks with considerable pollution of the environment.

WO-2011/1499948 describes biodegradable tear-off lenses which are however complex to make and therefore very expensive.

US-5163185 suggests a roller device comprising a mobile transparent film applied to a helmet visor. Said film accumulates in a side roller. There is no dispersion of material but the device is complex to apply and has a non-negligible weight, especially in competitions.

FR-3003730 discloses a viewing device for off-road activities, comprising a visor, at least a package of tear-off lenses mountable on a lens of the visor, retaining means of the visor and a retaining thread reversibly associated with the visor and with the retaining means of the visor.

It is the object of the present invention to make a viewing device with tear-off lenses which prevents tear-off lens dispersion after having been torn off.

It is a further object of the present invention that said device is simple and light-weight.

It is a yet further object of the present invention that said device is cost-effective and easily associated with competition helmets.

According to the invention, said and further objects are achieved by a viewing device for off-road activities as defined in claim 1.

Advantageously, the tear-off lens removed from the package does not fall to the ground because the retaining thread holds it hanging.

The removed tear-off lens uncouples from the visor and is able to slide along the retaining thread in a lateral position which does not obstruct the rider.

Advantageously, at the end of the race, the tear-off lenses removed and hanging on the retaining thread can be decoupled from the retaining thread and disposed of according to environmental regulations.

The retaining thread, therefore, acts both as a guide and retaining means for the tear-off lenses removed by the rider.

These and other features of the present invention will be more apparent from the following detailed description of a practical embodiment thereof, shown by way of non-limiting example in the accompanying drawings, in which:
figure 1 shows a perspective view of a goggles with tear-off lens during the removal of a tear-off lens;
figure 2 shows a perspective view of the goggles at the end of the removal of the tear-off lens;
figure 3 shows a section view of an attachment band of a retaining thread of the removed tear-off lens.

A viewing device comprises a goggles 1, a package 2 of tear-off lenses 3 with a tab 12 and a retaining thread 4.

The goggles 1 consists of an elastic band 7 and a visor 5 which is provided with a lens 6 on which the package 2 can be mounted.

There are two attachments 8 on the lens 6 in lateral position.

The package 2 provides two lateral through-holes 9 able to overlap said attachments 8. A plurality of packages 2 which consequently overlap, can be fitted on the attachments 8.

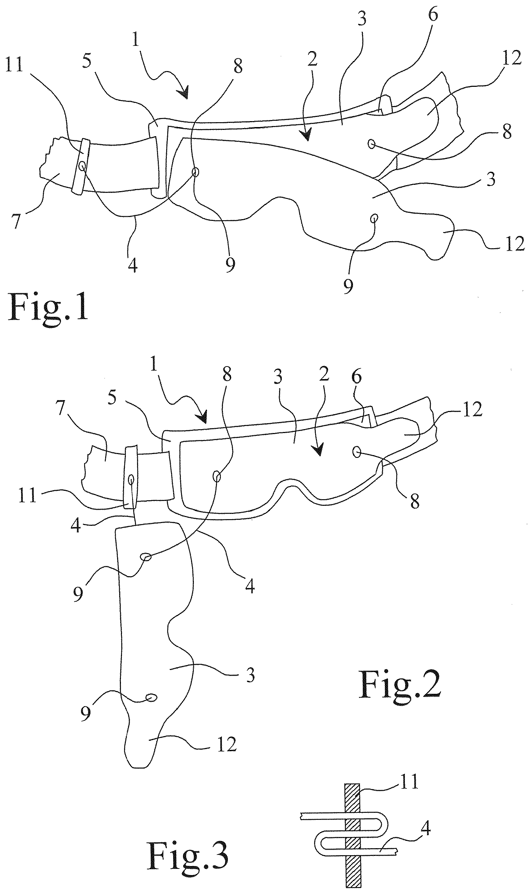

At a first end, the retaining thread 4, preferably made of nylon, provides an attachment pin able to couple reversibly with one of the attachments 8 of the lens 6. At a second end, the retaining thread 4 is able to be associated with a ring band 11 fixed to the elastic band 7. In particular, the retaining thread 4 runs through the ring band 11 three times, as shown in figure 3, to ensure the hold of the coupling during a competition, e.g. a motocross competition.

The simple functionality of the device can be understood by observing figures 1 and 2.

Once at least one package 2 has been secured to the visor 5 by engaging the lateral through-holes 9 of the tear-off lens 3 with attachments 8 of the lens 6, the attachment pin of the retaining thread 4 is connected to one of the attachments 8 of the lens 6. The other end of the retaining thread 4 is attached to the ring band 11.

The attachments 8 are substantially cylinder-shaped so that the lateral through-holes 9 of the tear-off lens 3 are crossed by said attachments 8 with slight interference which allows the package 2 to be positioned on the lens 6 of the visor 5.

Worn the goggles 1, with the band 7 gripping on the helmet, when the outermost tear-off lens 3 is fouled, the rider grips the corresponding tab 12 and tears said tear-off lens 3 from the package 2.

Advantageously, the tear-off lens 3 removed from the package 2 does not fall to the ground because the retaining thread 4 holds it hanging.

The removed tear-off lens 3 uncouples from the attachment 8 and is able to slide along the retaining thread 4 in a lateral position which does not obstruct the rider.

The operation is repeatable for all tear-off lenses 3 of the package 2 which accumulate hanging on the retaining thread 4.

The tear-off lenses 3 removed from the visor 5 are very light and therefore their accumulation does not create any problem for the rider.

Advantageously, at the end of the race, the removed tear-off lenses 3 hanging on the retaining thread 4 can be decoupled from the retaining thread 4 and disposed of according to environmental regulations.

The retaining thread 4, therefore, acts as both a guide and retaining means for the tear-off lenses 3 removed by the rider.

The retaining thread 4 can be reused because it can be decoupled from the visor 5 with which one or more new packages 2 of tear-off lens 3 can be associated.

In an example not forming part of the invention, instead of the goggles 1, the device may comprise a helmet with integrated visor 5: the retaining thread 4 is associated with the visor 5 and directly with the helmet, thus obtaining the same identical function of accumulation means.

The helmet and the elastic band 7 of the goggles 1 are examples of retaining means of the visor 5 with which one end of the retaining thread 4 can be associated to obtain the innovative retaining function described herein.

Alternatively, the visor 5 may have a single attachment 8 as well as the package 2, the adherence of the latter to the lens 6 being ensured by adhesive means.

## Claims

1. Viewing device for off-road activities, comprising a visor (5), at least a package (2) of tear-off lenses (3) mountable on a lens (6) of the visor (5), and retaining means of the visor (5), wherein the tear-off lenses (3) have superimposable through holes (9), wherein the device further comprises a retaining thread (4) reversibly associated with the visor (5) and with the retaining means of the visor (5), wherein the tear-off lenses (3) after being torn off are able to be retained by the retaining thread (4) sliding along it by means of the through holes (9),
the device further comprising goggles (1) provided with said visor (5), wherein the retaining means is an elastic band (7) of the googles (1),
**characterized in that** at one end the retaining thread (4) is able to be associated with a ring band (11) fixed to the elastic band (7).

2. Device according to claim 1, **characterized in that** the visor (5) comprises at least one attachment (8) to which at least a series of through holes (9) of the tear-off lenses (3) of the package (2) are able to be coupled, and an attachment pin associated with one end of the retaining thread (4).

3. Device according to claim 1, **characterized in that** the retaining thread (4) passes through the ring band (11) three times.

## Patentansprüche

1. Sichtvorrichtung für Aktivitäten im Gelände, umfassend ein Visier (5), zumindest ein Paket (2) Abreißgläser (3), die auf einer Linse (6) des Visiers (5) anbringbar sind, und ein Halteelement des Visiers (5), wobei die Abreißgläser (3) überlagerbare Durchgangslöcher (9) aufweisen, wobei die Vorrichtung weiter einen Haltefaden (4) umfasst, der reversibel mit dem Visier (5) und mit dem Halteelement des Visiers (5) verbunden ist, wobei die Abreißgläser (3) nach dem Abreißen durch den Haltefaden (4) gehalten werden können, der mittels der Durchgangslöcher (9) an ihm entlang gleitet,
wobei die Vorrichtung weiter eine mit dem Visier (5) versehene Brille (1) umfasst, wobei das Halteelement ein elastisches Band (7) der Brille (1) ist,
**dadurch gekennzeichnet, dass** der Haltefaden (4) an einem Ende mit einem an dem elastischen Band (7) befestigten Ringband (11) verbunden werden kann.

2. Vorrichtung nach Anspruch 1, **dadurch gekennzeichnet, dass** das Visier (5) zumindest eine Befestigung (8) umfasst, an die mindestens eine Reihe von Durchgangslöchern (9) der Abreißgläser (3) des Pakets (2) gekoppelt werden kann, und einen Befestigungsstift, der mit einem Ende des Haltefadens (4) verbunden ist.

3. Vorrichtung nach Anspruch 1, **dadurch gekennzeichnet, dass** der Haltefaden (4) das Ringband (11) dreimal durchläuft.

## Revendications

1. Dispositif de visualisation pour activités hors-route, comprenant une visière (5), au moins un ensemble (2) de verres détachables (3) pouvant être monté sur un verre (6) de la visière (5), et le moyen de retenue de la visière (5), dans lequel les verres détachables (3) ont des trous débouchants (9) superposables, dans lequel le dispositif comprend en outre un fil de retenue (4) associé, de manière réversible, avec la visière (5) et avec le moyen de retenue de la visière (5), dans lequel les verres détachables (3), après avoir été détachés, peuvent être retenus par le fil de retenue (4) coulissant le long de ce dernier au moyen des trous débouchants (9),
le dispositif comprenant en outre un masque (1) prévu avec ladite visière (5), dans lequel le moyen de retenue est une bande élastique (7) du masque (1),
**caractérisé en ce qu'**au niveau d'une extrémité, le fil de retenue (4) peut être associé avec une bande annulaire (11) fixée à la bande élastique (7).

2. Dispositif selon la revendication 1, **caractérisé en ce que** la visière (5) comprend au moins une fixation (8) à laquelle au moins une série de trous débouchants (9) des verres détachables (3) de l'ensemble (2) peut être couplée, et une broche de fixation associée à une extrémité du fil de retenue (4)

3. Dispositif selon la revendication 1, **caractérisé en ce que** le fil de retenue (4) passe à travers la bande annulaire (11) trois fois.
